Europäisches Patentamt

European Patent Office

Office européen des brevets

(11) Publication number: **0 409 372 A1**

## EUROPEAN PATENT APPLICATION

(21) Application number: 90302066.7

(22) Date of filing: 27.02.90

(51) Int. Cl.⁵: **A61M 25/01, A61B 17/22**

(30) Priority: 21.07.89 US 410623

(43) Date of publication of application:
23.01.91 Bulletin 91/04

(84) Designated Contracting States:
BE DE FR GB LU NL

(71) Applicant: INTERVENTIONAL TECHNOLOGIES INC
4949 Viewridge Avenue
San Diego California 92123(US)

(72) Inventor: Gomringer, Gary
10880 Starvation Mountain Road
Escondido, California 92025(US)

(74) Representative: Coxon, Philip et al
Eric Potter & Clarkson St. Mary's Court St.
Mary's Gateate
Nottingham NG1 1LE(GB)

(54) Telescopic control unit for an atherectomy device.

(57) A telescopic control unit (10) for an atherectomy device to incrementally cut a passageway (52) through obstructive tissue (46) in arteries comprises a cylindrical-shaped hollow housing (12) which has a tube-shaped telescoping insert (16) coaxially aligned within an interior chamber of the housing. The telescoping insert is held in place with respect to the housing by threadably engaging a ring-shaped cap (14) to the housing which causes an O-ring (32) to urge against the insert and hold it securely in place. A tube-shaped sheath or guiding catheter (18) for an atherectomy cutter is connected to the distal end of the insert. A cutter assembly comprised of a torque tube (22) and cutter (50) is slidably carried inside the guiding catheter. The torque tube is operably connected to a handpiece drive unit (66) coupled to the control unit to rotatably drive the cutter. Selective control of telescoping insert incrementally advances the handpiece drive unit, and then the cutter, deeper into the artery. The obstructive tissue cuttings are removed from the artery by a suction system which is attached at the proximal end of the housing. The housing device is also equipped with a port (26) for injecting medicinal fluids therethrough to aid in the operation.

Fig. 2

EP 0 409 372 A1

This invention relates generally to devices for controlling the removal of obstructive tissue and in particular to devices for controlling the removal of obstructive tissue from arteries in humans. More particularly, the present invention pertains to the control unit of an atherectomy device which is used to selectively change the effective length of the device and extend its rotating cutter for drilling a passageway through obstructive tissue in the arteries. This invention is particularly, but not exclusively, useful for incrementally drilling a passageway through plaque which obstructs the flow of blood through arteries.

As is well known in the medical field, blockages in coronary arteries due to the accumulation of plaque on the arterial wall will cause partial or complete stoppage of the flow of blood through these arteries. Such a condition can cause heart attacks. Similarly, when peripheral arteries are blocked with such obstructive tissue, other serious problems can result. For example, obstructive tissue in the arteries of the legs may lead to amputation of feet or limbs. Also, strokes can be caused by the build up of plaque in arteries of the head and neck, and hypertension has been linked to obstructive tissue in the renal arteries. Unquestionably, there is a need either to avoid the build up of plaque in the arteries or to provide a way to increase the flow of blood through arteries in which plaque build up has already occurred.

One method which has been used to increase the flow of blood through blocked arteries is the well known angioplasty procedure. During an angioplasty procedure, a catheter is used to place a deflated balloon in apposition to the obstructive tissue within a blocked artery. The balloon is then inflated to cause the arterial wall to expand. This expansion tears and compresses the plaque to reopen the vessel lumen for increased blood flow. One such device used in the angioplasty procedure is disclosed in United States Patent No 3,467,101. Unfortunately, during an angioplasty procedure, the integrity of the arterial wall may be compromised and damaged when it is stretched by inflating the balloon. Consequently, the risk is increased that future physiological problems can occur because of the damaged arterial wall. Moreover, the angioplasty procedure cannot remove plaque from the artery. Thus, since the obstructive tissue continues to remain in the artery, a recurrence of the blockage (restenosis) has been a frequent problem.

To overcome many of the problems associated with the angioplasty procedure, atherectomy procedures have been developed which cut passageways through the obstructive tissue in a blocked artery. Some atherectomy devices collect and remove the obstructive tissue from the artery. Others do not. One device which does remove excised tissue is disclosed in co-pending patent United States Patent Application Serial No. 123,713 and United States Patent Application Serial No. 213,691.

In addition to recovering excised tissue, the atherectomy system and method described in the above referenced patent application Serial No. 123,713 provides a telescopically sliding means within the handpiece (the drive unit) which can provide as much as 2.5 cm of telescopic advancement or retraction of the rotatable cutter during an excision of obstructive tissue. Further, this sliding means within the handpiece allows precise and tactile control of the cutting process through selective variation of the length of catheter, and consequently, the location of the cutter within the artery. This may, perhaps, be best understood by using the site of entry into an artery as a reference point. The obstructive tissue of interest in an arterial tree will lie at some arbitrary but fixed distance from this entry site. Hence, some arbitrary length of catheter will be required to reach any given lesion in the body. The length of the catheter, from the entry site to the obstruction, is manually fed into the artery over a guide wire using fluoroscopy for control. The proximal end of the catheter and cutter assembly is then mechanically coupled to the handpiece (ie. drive unit) and the handpiece is further coupled to the entry site. When these cited system components have been coupled, the total length of the system, both within and without the artery, from the obstruction to the handpiece has been defined. In order to remove the obstruction, an additional length of catheter which is equal to the lesion length must still be advanced into the artery. Normally, in treating short focal lesions (obstructions), this additional length is available by extending the telescopic sliding means from within the handpiece. Unfortunately, diffuse obstructions can exceed 2.5 cm in length. Thus some device is needed which will permit even further advancement of the catheter and cutter into the obstruction.

It is an object of this invention to provide an improved device for controlling the removal of obstructive tissue.

According to one aspect of this invention there is provided a device for controlling the removal of obstructive tissue comprising:
a substantially cylindrical-shaped hollow housing;
a tube-shaped insert coaxially aligned and slidingly disposed within said housing, said insert having its distal end connectable to a sheath; over a rotatable torque tube and
a releasable insert holding means mounted on said housing for selectively moving said insert within said housing.

The device may be in the form of a telescopic control unit for positioning a support sheath over a rotatable torque tube and its associated atherectomy cutter.

According to another aspect of this invention there is provided a telescopic control unit for positioning a support sheath over a rotatable torque tube and its associated atherectomy cutter which comprises:

a substantially cylindrical-shaped hollow housing;

a ring-shaped cap threadably engageable with said housing having a concentric hole through its top; and

a tube-shaped insert slideably engaged with said hole of said cap and coaxially positioned within the interior of said housing, said insert having its distal end connectable to said support sheath.

Preferably, the unit further comprises an O-ring positioned between said housing and said cap which fixedly holds said insert relative to said housing when said cap is threadably engaged with said housing.

The housing may have interior threads at a proximal end thereof for engagement with a suction means.

The housing may have a port in said housing for injecting medicinal fluids into said housing.

The insert may be flanged at a proximal end thereof for providing longitudinal alignment of said insert inside of said housing when said insert is moved in and out of said housing.

The housing, the cap and the insert may be made of polycarbonate. The O-ring may be made of an elastomeric material.

The cap may have a grooved outer surface for enabling manual engagement and disengagement.

The insert may be connected to said sheath by threadable engagement. Alternatively, the insert may be connectable to said sheath by a Luer fitting.

According to another aspect of this invention there is provided a method for incrementally cutting through obstructive material in a conduit by selectively displacing a support sheath relative to a cutter with a telescopic control unit which comprises the steps of:

(A) Positioning said cutter to come into contact with said obstructive material;

(B) Cutting said obstruction by rotating said cutter;

(C) Displacing said sheath in a proximal direction relative to said cutter to a predetermined position;

(D) Rotating said cutter to drill a passageway through said obstruction proportional to the distance of displacement in step (C);

(E) Repeating step (C) and step (D) until a continuous passageway is drilled through said obstruction; and

(F) Withdrawing said obstructive tissue, said cutter, and said sheath from said conduit. The method can be used for cutting through obstructive tissue in arteries of living beings.

A preferred embodiment of the atherectomy device control unit of the present invention comprises a cylindrical-shaped hollow housing having an interior chamber that extends longitudinally between the proximal end and the distal end of the housing. A ring-shaped cap having a hole through its top may be threadably engaged to the distal end of the housing. A ledge may be formed within the interior chamber of the housing at the distal end thereof to establish a gap between the ledge and the cap wherein an elastic O-ring is positioned.

A tube-shaped insert having a flange at its proximal end may be coaxially aligned within the housing and positioned to slide through the hole of the cap. When so positioned, the flange may slide along the inside wall of the chamber to keep the insert longitudinally aligned within the housing. As the cap is screwed tightly onto the distal end of the housing, the O-ring may be compressed between the cap and the ledge thereby to distort the O-ring. The distorted O-ring may squeeze against the insert to longitudinally stationarily hold the insert within the housing. Loosening the cap on its threads may relax the O-ring to reduce the friction on the tube-shaped insert so the insert may be slidingly telescoped, as desired, within the outer housing. Selective control of this longitudinal relationship between the tube-shaped insert and the outer housing may allow the overall length of the telescopic control unit to be changed as desired. This selective variability in the length of the telescopic control unit outside the body, in turn, allows for a corresponding advancement of the cutter disposed inside the artery.

Suitable mechanical means (eg. Luer fittings) may be provided on both proximal and distal ends of the telescopic control unit for coupling, respectively, with the arterial entry site and the handpiece of the device. A port, which may be fitted with suitable tubing and a Luer stopcock, may extend through the housing into the interior chamber of the control unit to allow for the infusion of fluids around the catheter and cutter assembly of the device and into the artery of the patient during the conduct of a procedure.

The principle of operation of the telescopic control unit of the present invention is the same without regard to a particular application or the particular system components being used. In preparing for an atherectomy procedure with the system, entry into an artery may be made percutaneously. The entry site may be fitted with a suitable sheath or guiding catheter depending upon the nature (ie. peripheral or coronary) of the procedure, the guiding catheter may extend into the artery from the entry site to the obstruction. A mechanical fitting on the proximal end of the sheath or catheter may serve as a relatively stable reference point and as an interface for other system components. After a guide wire has been put into place with respect to a lesion in an artery, a catheter with a cutter attached to its distal end may be first threaded through the lumen of the telescopic control unit, thence over the guide wire and, finally through a sheath to a peripheral lesion. Alternatively, the catheter can be threaded through a guiding catheter to a coronary lesion. The distal end of the fully extended telescopic control unit may then be suitably coupled to the external mechanical fitting at the arterial entry site to fixedly hold the distal end of the telescopic control unit with respect to the sheath or guiding catheter. This may also hold the control unit in a fixed relationship to the obstruction within the artery of the patient. As so connected, the telescopic control unit may become an extension of the sheath or guiding catheter. The catheter and cutter assembly, on the other hand, may be mechanically fixed to a sliding tubular member within the handpiece and drive unit of the device. This sliding tubular member can be advanced or retracted approximately 2.5 cm within the handpiece to effectively move the catheter and cutter assembly through the sheath with respect to the handpiece and drive unit. Thus, with the handpiece fixed in its positional relationship to the arterial entry site, the catheter and cutter can be extended into the artery by approximately 2.5 cm with this arrangement.

With the control unit appropriately coupled and positioned between the stationary entry site and the handpiece, it will be appreciated by the skilled artisan that, by shortening the control unit, the handpiece can be moved closer to the entry site. Accordingly, while the control unit is held in its extended configuration, the cutter can be advanced approximately 2.5 cm by manipulation of the handpiece to move the sliding tubular member in the handpiece. The control unit can then be incrementally shortened to draw the hand piece closer to the entry site and reposition the sliding tubular member in the handpiece for subsequent advancement of the cutter up to another approximately 2.5 cm. Thus, progressively shortening the length of the catheter and cutter assembly disposed outside the body, by collapsing the telescopic control unit into itself, causes an equal lengthening of the catheter and cutter assembly inside the body and extends its effective range of cutting. This procedure can be repeated as required.

A vacuum source can be engaged with the proximal end of this system to withdraw tissue cut from the arterial lesion during an atherectomy procedure. Also, a port may extend into the interior chamber of the telescopic control unit to facilitate infusion of fluids during the atherectomy procedure.

Accordingly, an advantage of the present invention is that it provides a probe for controlling the cutter of an atherectomy device as it cuts a passageway through obstructive tissue in an artery. Another advantage of the present invention is that it provides a probe for an atherectomy device which facilitates the incremental advancement of a rotating cutter and its associated torque tube beyond the support sheath within a determined safety range while moving only the sheath. Yet another advantage of the present invention is that it provides a probe for an atherectomy device which is easily manufactured, easy to operate, and relatively inexpensive.

Reference is now made to the accompanying drawings in which:

Figure 1 is a perspective view of one preferred embodiment of the telescopic control unit of the present invention shown in combination with an atherectomy guiding catheter;

Figure 2 is a cross-sectional view of the telescopic control unit of Figure 1 as seen along the line 2-2 in Figure 1;

Figures 3A, 3B, 3C and 3D are cross-sectional views of an atherectomy cutter in association with a coronary guiding catheter which illustrate the incremental advancement of the cutter as it cuts through obstructive tissue in an artery under control of the telescopic control unit of the present invention; and

Figures 4A, 4B, 4C and 4D are diagrammatic side views partly in cross section of the telescopic control unit, guiding catheter and cutter illustrating operation of the telescopic control unit to advance the cutter.

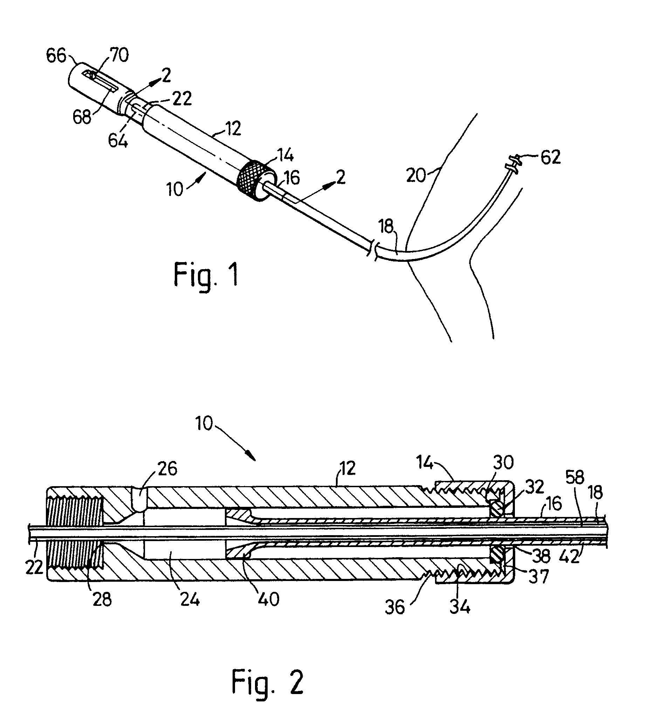

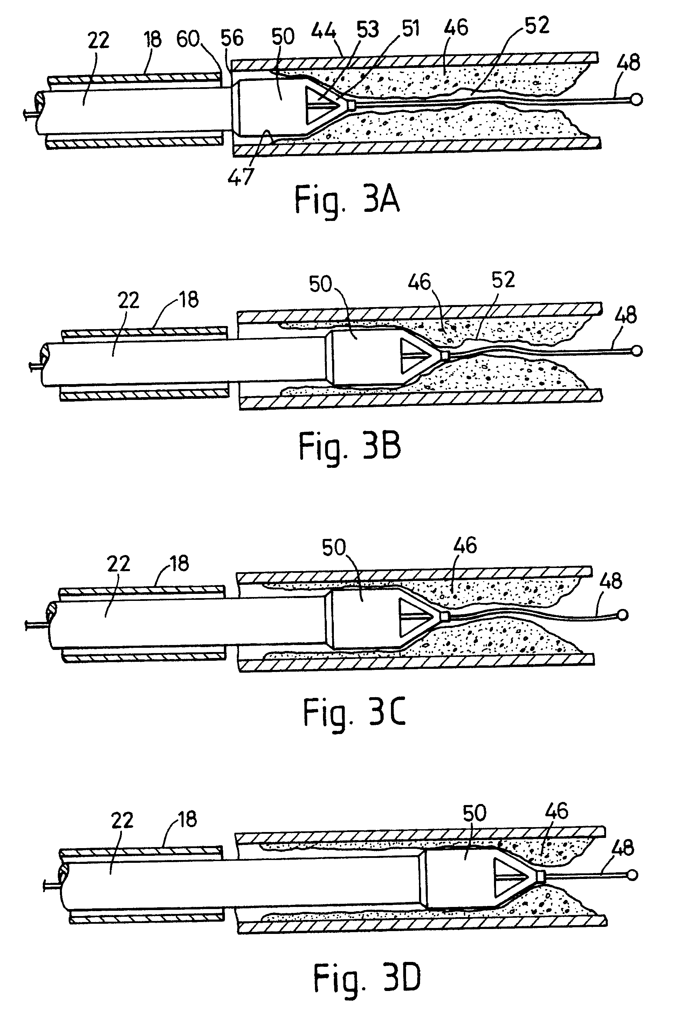

Referring initially to Figure 1, the present invention is shown in its intended environment. In Figure 1, the telescopic control unit, generally designated 10, is shown connected to a substantially tubular sheath or guiding catheter 18 which is inserted into an artery of a patient 20. A hollow torque tube 22 has a hollow atherectomy cutter 50 affixed to the distal end thereof to form a catheter cutter assembly (see Figure 3A). Cutter 50 has blades 51 and openings 53 for collecting excised tissue, which may be drawn out and removed by a vacuum applied to hollow torque tube 22. Torque tube 22 and cutter 50 are introduced into the patient 20 through control unit 10 and guiding catheter 18. The cutter 50 on torque tube 22 can thus be used to cut through obstructive tissue within the artery of the patient 20, as will be described further below.

As shown in Figure 1, control unit 10 comprises a substantially cylindrical-shaped hollow housing 12 having a ring-shaped cap 14 threadably engaged on the distal end thereof. Cap 14 can be manipulated to retain a tube-shaped insert 16 at selected positions within housing 12. As can be appreciated, as insert 16 is telescopically retracted into housing 12, torque tube 22 and cutter 50 can be correspondingly inserted more deeply into the artery of patient 20. A port 26 extends through the wall of housing 12 into the interior chamber 24 of control unit 10. Port 26 is adapted to be fitted with suitable PVC tubing and a Luer stop-cock (not shown) for introducing fluids into interior chamber 24 as will be more fully described below.

Also shown in Figure 1 is a handpiece drive unit 66 which provides the rotating power to torque tube 22 to rotate cutter 50. Housed within handpiece drive unit 66 is a drive tube or shaft 68 connected to torque tube 22. Drive tube 68 is telescopically slidable and has a tab 70 to manually advance rotating drive tube 68 a predetermined distance, e.g. approximately 2.5 cm. Proximal end 64 of torque tube 22 is operably coupled to slidable drive tube 68. It is typically advanced by the physician who is operating the cutter, by using his or her thumb to slidably move tab 70. The physician can then controllably extend or retract the cutter 50 in an artery 44 as required during the particular procedure, as more fully explained below.

The arrangement and cooperation of the components of telescopic control unit 10 can perhaps be best seen with reference to Figure 2. Specifically, for the preferred embodiment of telescopic control unit 10, housing 12 is preferably formed of a plastic such as polycarbonate or acrylobutylstyrene (ABS). An interior chamber 24 extends longitudinally between the proximal end and the distal end of housing 12. The proximal end of housing 12 forms an opening 28 through which torque tube 22 can be inserted.

Still referring to Figure 2, it can be seen that a ledge 30 is formed at the distal end of housing 12. A ring-shaped cap 14, having a hole 38 therethrough and provided with interior threads 34, is threadably engaged to a plurality of threads 36 formed on the exterior of the distal end of housing 12. Cap 14 is preferably made of a relatively rigid plastic such as polycarbonate PVC or polyester. Cap 14 has a knurled or grooved outer surface, as shown in Figure 1, to facilitate manual engagement and disengagement thereof. As shown in Figure 2, a gap 37 is formed between ledge 30 of housing 12 and cap 14 when cap 14 is connected to housing 12. An O-ring 32, which is made of an elastomer such as rubber, is positioned within this gap 37.

A tube-shaped insert 16 having a flange 40 at its proximal end is coaxially aligned within housing 12. Insert 16 is positioned to slide telescopically within chamber 24 of housing 12 and through hole 38 in cap 14. Insert 16 is preferably made of PVC. When insert 16 is positioned within chamber 24 of housing 12 with a portion thereof extending through hole 38, flange 40 abuttingly slides along the inside wall of chamber 24 and keeps insert 16 longitudinally aligned within housing 12. In addition, flange 40 eventually abuts against O-ring 32 when insert 16 is positioned in its extreme distal direction, thereby preventing complete withdrawal of insert 16 from chamber 24. The distal end 42 of insert 16 is connected to proximal end 58 of sheath or catheter 18 by any method well known in the art. For example, a well known Luer fitting or some means for threaded or snapped engagement of end 58 to end 42 can be used. As is well known to the skilled artisan, such other methods may include a threaded engagement or Luer fitting (not shown).

As can be appreciated, when O-ring 32 is in a relaxed position, O-ring 32 has a substantially annular cross-sectional shape. Significantly, in its relaxed position, O-ring 32 does not prevent insert 16 from sliding along its longitudinal axis. Rather, when O-ring 32 is relaxed, insert 16 may slide freely in and out of housing 12 through cap 14 and O-ring 32. As cap 14 is screwed onto housing 12, however, O-ring 32 is compressed between cap 14 and ledge 30 of housing 12. This causes O-ring 32 to become distorted and to squeeze against insert 16 to hold it in a substantially fixed position within housing 12. Thereafter, when cap 14 is unscrewed slightly from housing 12, elastic O-ring 32 returns to its original shape, and insert 16 can again be easily moved with respect to housing 12 along its longitudinal axis.

As previously stated, housing 12 is also provided with a port 26 which extends through housing 12 and into chamber 24. Port 26 is required for injecting medicinal fluids, as needed, into chamber 24. Fluids introduced into chamber 24 are transmitted through insert 16 and sheath 18 into the artery 44 of patient 20 to aid in an atherectomy procedure.

OPERATION

It is to be appreciated that in undertaking an atherectomy procedure to remove obstructive tissue from an artery, a number of preparatory steps are involved. Although there may be minor differences in equipment and practice involved in the various types of procedures, the principles of operation of the present invention are essentially the same. For purposes of illustration of operation of the telescopic control unit 10, the procedure is assumed to be a coronary atherectomy.

The main preparatory steps are now described as follows:

Initially, a percutaneous entry at entry site 62 into the arterial system is made. The entry site 62 is then intubated with guiding catheter 18 and its distal or leading tip 60 is positioned, under flouroscopic control, at the ostium 56 (entry) of the coronary artery 44 of interest. The distal end 42 of insert 16 of telescopic control unit 10 is then coupled to proximal end 58 of guiding catheter 18. Next, guide wire 48 is threaded through telescopic control unit 10, then into and through the guiding catheter 18, and thence to, through, and somewhat beyond the existing lumen of the obstructive tissue 46. The cutter assembly comprised of the cutter 50 with torque tube 22 is then slidably advanced along guide wire 48, through the guiding catheter 18 to the mouth 47 of the obstructive tissue 46. The proximal end 64 of the torque tube 22 is then mechanically coupled to telescopically slidable drive tube 68 of the handpiece drive unit 66.

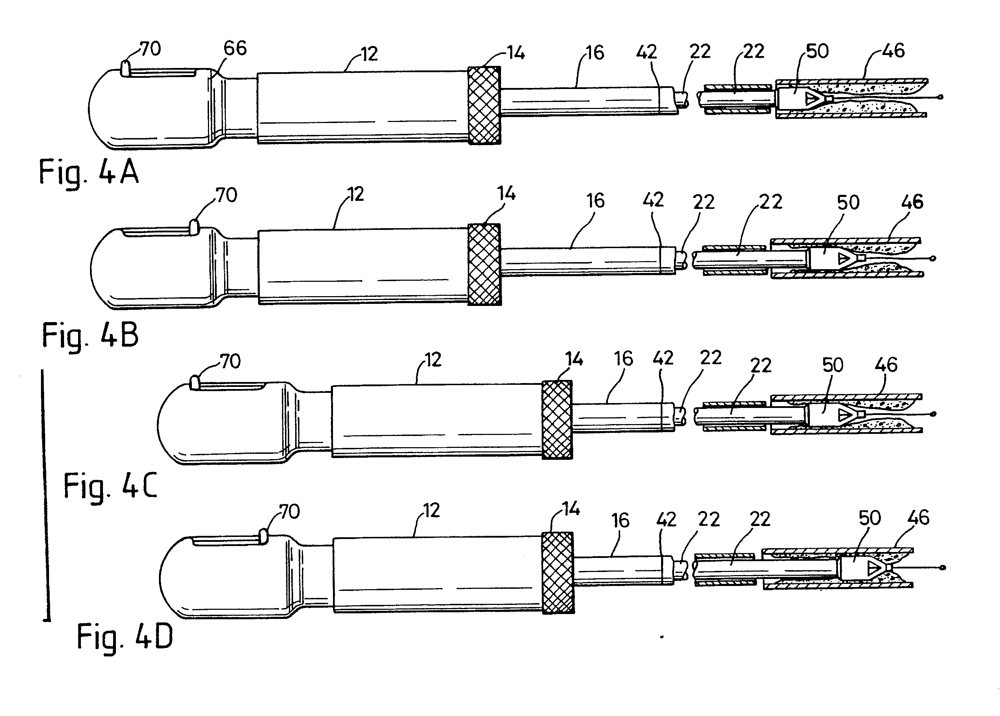

Figures 3A and 4A show that distal tip 60 of guiding catheter 18 has been placed at ostium 56 of artery 44. Figure 3A further shows guide wire 48 passing through lumen 52 of obstructive lesion or tissue 46. Torque tube 22 and cutter 50 are shown in place immediately adjacent to lesion 46. Also, telescopic control unit 10 is placed in a fully extended position with insert 16 tightly held in place in its fully extended position by cap 14 on housing 12.

For brevity, it will now be assumed that the entire atherectomy system, in all details, has been assembled as set forth above and is ready for operation. Torque tube 22 with attached cutter 50 is rotated by suitable means such as an electronic motor within handpiece drive 66. Rotating torque tube 22 with cutter 50 is then advanced by the physician using tab 70 to extend rotatable drive tube 68 in handpiece drive unit 66. This advances torque tube 22 through telescoping control unit 10, deeper into artery 44. This causes cutter 50 to advance into and cut tissue from obstructive lesion 46 forming passageway 52. Excised tissue is collected and removed from artery 44 by vacuum applied through openings 53 of cutter 50 via torque tube 22.

Figures 3B and 4B show cutter 50 when it has been advanced by the physician moving tab 70 one increment, e.g. approximately 2.5 cm, into lesion 46. This increment is the limit of travel provided by extending drive tube 68 of handpiece drive unit 66. This increment is typically less than the length of insert 16, as further explained below. As illustrated in Figure 3B and 4B, however, it can be seen that further advancement of cutter 50 is required to excise more tissue 46 to open lumen 52.

To obtain additional advancement capability using the telescopic control unit 10, reference is further made to Figures 3C and 4C. Knurled cap 14 is loosened on control unit body 12 so that control unit body 12 can be slidably advanced onto tube-shaped insert 16. Tube insert 16 is thus retracted within control unit body 12 for a distance of one increment, e.g. approximately 2.5 cm. This retractive motion is made while holding tube-shaped insert 16 and guiding catheter 18 motionless with respect to arterial entry site 62. In other words, retractive telescoping is done by sliding body 12 of control unit 10 toward patient 20 over tube insert 16 as shown in Figure 4C. Thus, while this retractive motion reduces the effective length of guiding catheter 18 between patient 20 and handpiece drive unit 66, it also allows handpiece drive unit 66 to move approximately 2.5 cm closer to patient 20.

At the same time, however, the relative position of torque tube 22 in artery 44 does not change since tube-shaped insert 16 of control unit 10 passes freely over torque tube 22. Telescopically sliding drive tube 68 of handpiece drive unit 66 is retracted 2.5 cm back into handpiece drive unit 66. The net effect of these operations, as outlined, is to place the handpiece drive unit 66 and tab 70 back in their original configuration. Then, torque tube 22 and cutter 50 can again be advanced into obstructive tissue 46 by an increment of up to approximately 2.5 cm by extending drive tube 68 as shown in Figure 4D. This in effect gives the physician up to approximately 2.5 cm more advancing capability to cut into obstructive tissue 46. This process may be repeated a number of times, as required, limited only by the absolute length of telescopic control unit 10.

While the particular telescopic control unit as herein shown and disclosed in detail is fully capable of obtaining the objectives and providing the advantages hereinbefore stated, it is to be understood that it is merely illustrative of the presently preferred embodiment of the invention and that no limitations are intended to the details of construction or design herein shown other than as defined in the appended claims.

**Claims**

1. A device for controlling the removal of obstructive tissue comprising

a substantially cylindrical-shaped hollow housing;

a tube-shaped insert coaxially aligned and slidingly disposed within said housing, said insert having its distal end connectable to said sheath, and

a releasable insert holding means mounted on said housing for selectively moving said insert within said housing.

2. A device according to claim 1 wherein said insert holding means comprises a ring-shaped cap threadably engageable with said housing for squeezing an O-ring positioned between said cap and said housing against said insert when said cap is engaged with said housing.

3. A device according to claim 2 wherein said cap has a concentric hole for slideably holding said insert and for coaxially aligning said insert inside said housing.

4. A device according to Claim 2 or 3 wherein said cap has a grooved outer surface for enabling manual engagement and disengagement.

5. A device according to Claim 2, 3 or 4 wherein said housing , said cap and said insert are made of polycarbonate.

6. A device according to any of Claims 2 to 5 wherein said O-ring is made of an elastomeric material.

7. A device according to any preceding claim wherein said insert is flanged at a proximal end thereof for providing longitudinal alignment of said insert with said housing.

8. A device according to any preceding claim wherein said housing has interior threads at a proximal end thereof for threadable engagement with a suction means.

9. A device according to any preceding Claim further comprising a port in said housing for injecting medicinal fluids inside of said housing.

10. A device according to any preceding Claim wherein said insert is connected to said sheath by threadable engagement.

11. A device according to any preceding Claim wherein said insert is connectable to said sheath by a Luer fitting.

12. A method for incrementally cutting through obstructive material in a conduit by selectively displacing a support sheath relative to a cutter with a telescopic control unit which comprises the steps of:

(A) Positioning said cutter to come into contact with said obstructive material;

(B) Cutting said obstruction by rotating said cutter;

(C) Displacing said sheath in a proximal direction relative to said cutter to a predetermined position;

(D) Rotating said cutter to drill a passageway through said obstruction proportional to the distance of displacement in step (C);

(E) Repeating step (C) and step (D) until a continuous passageway is drilled through said obstruction; and

(F) Withdrawing said obstructive material, said cutter, and said sheath from said conduit.

13. A method according to Claim 12 wherein positioning said cutter to come into contact with said obstructive material comprises the steps of:

(A) inserting a guide wire through a tip of said cutter;

(B) sliding said guide wire through and past said obstructive material of said conduit;

(C) positioning said cutter inside said sheath; and

(D) sliding said sheath and said cutter through said conduit to a position adjacent to said obstructive tissue.

14. A method according to Claim 12 or 13 wherein displacing of said sheath to a predetermined position by manipulating a cylindrical-shaped hollow housing, a ring-shaped cap and a tube-shaped insert of said telescopic control unit comprises the steps of:

(A) disengaging said ring-shaped cap from said cylindrical-shaped hollow housing by counterclockwise rotation;

(B) sliding said tube-shaped insert into said housing to a predetermined position; and

(C) engaging said cap with said housing by clockwise rotation to prevent movement of said insert relative to said housing along the longitudinal axis thereof.

15. A method according to Claim 14 further comprising the steps of:

(A) Injecting fluids through said housing during operation to enable use of said fluids in said cutting procedure;

(B) Attaching a suction means to the proximal end of said housing for removing said obstructive material cuttings; and

(C) Withdrawing said guide wire from said conduit after said cutting procedure.

Fig. 1

Fig. 2

Fig. 3A

Fig. 3B

Fig. 3C

Fig. 3D

9

Fig. 4A

Fig. 4B

Fig. 4C

Fig. 4D

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 321 132 (BAXTER)<br>* Column 5, lines 24-26; column 7, lines 19-13; figures 2,8 *<br>– – – | 1-6 | A 61 M 25/01<br>A 61 B 17/22 |
| Y | | 7-15 | |
| Y | EP-A-0 289 319 (ANGIOMEDICS)<br>* Column 5, lines 60-65; column 4, lines 50-56; figure 1 *<br>– – – | 7-15 | |
| A | US-A-4 688 554 (HABIB)<br>* Figures 1, item 34; column 4, lines 48-56 *<br>– – – | 1-6 | |
| A | EP-A-0 177 782 (ADVANCED TECHNOLOGY LABS., INC.)<br>* Figure 1; page 4, lines 24-26 *<br>– – – – – | 9 | |

| | TECHNICAL FIELDS SEARCHED (Int. Cl.5) |
|---|---|
| | A 61 B<br>A 61 M |

The present search report has been drawn up for all claims

| Place of search | Date of completion of search | Examiner |
|---|---|---|
| The Hague | 23 October 90 | BARTON S.A. |